Europäisches Patentamt

European Patent Office

Office européen des brevets

(19)

(11) EP 0 692 714 A1

## (12) DEMANDE DE BREVET EUROPEEN

(43) Date de publication:
17.01.1996 Bulletin 1996/03

(51) Int Cl.$^6$: G01N 33/46, G01N 21/89

(21) Numéro de dépôt: 95401649.9

(22) Date de dépôt: 07.07.1995

(84) Etats contractants désignés:
AT DE DK IT SE

(30) Priorité: 12.07.1994 FR 9408630

(71) Demandeur: CENTRE TECHNIQUE DU BOIS ET
DE L'AMEUBLEMENT
F-75012 Paris (FR)

(72) Inventeurs:
• Vogrig, Raphäel J.
F-54000 Nancy (FR)
• Karpp, Bernard F.
F-57470 Hombourg-Haut (FR)

(74) Mandataire: Thévenet, Jean-Bruno et al
F-75340 Paris Cédex 07 (FR)

(54) Procédé et dispositif de reconnaissance de particularités géométriques de pièces parallélépipédiques de section polygonale

(57) Le procédé consiste à :

a) disposer une source laser (21) projetant un faisceau plan de rayonnement de manière à éclairer de face une première face (3) sans éclairer une face adjacente (1),

b) disposer une caméra vidéo linéaire couleur (11) en regard de la face adjacente (1) considérée, de telle sorte qu'elle observe une ligne perpendiculaire à l'axe longitudinal de la pièce (5), dans le plan du faisceau de rayonnement laser, de manière à observer à la fois la face adjacente (1) et la projection de la première face (3) sur la face adjacente (1),

c) acquérir simultanément les trois signaux couleur R, V, B issus de la caméra (11),

d) traiter en temps réel les signaux R, V, B pour fournir une succession de pixels adjacents et associés selon l'appartenance de leurs composantes R, V, B à des domaines particuliers de l'espace R, V, B identifiés par des labels,

e) produire un déplacement longitudinal relatif de la pièce à analyser par rapport à la caméra (11) et à la source laser (21) pour observer l'ensemble de la face adjacente (1) et la projection de la première face (3) sur la face adjacente (1), et

f) reconnaître les particularités géométriques (8) de la première face (3) à partir de l'identification des pixels correspondant à un domaine particulier de l'espace R, V, B identifié par un label correspondant à l'éclairage monochromatique de la source laser

(21).

FIG.6

## Description

La présente invention concerne un procédé et un dispositif de reconnaissance de particularités géométriques de pièces parallélépipédiques de section polygonale comportant au moins une face secondaire adjacente à une face principale, l'angle maximum défini entre la face principale considérée et ladite face secondaire adjacente étant compris entre 0° et 90°.

On connaît déjà divers systèmes d'observation d'objets, tels que des pièces de bois, à l'aide de caméras vidéo couleur matricielles ou linéaires. Ces systèmes d'inspection automatique d'objets servent à repérer des défauts d'aspect tels que des défauts de coloration ou de structure sur des faces planes des objets observés afin de permettre ensuite par exemple un triage qualitatif de ces objets.

Chaque face plane d'un objet est, selon les systèmes d'observation connus, examinée à l'aide d'une caméra vidéo couleur associée à un système d'éclairage. Un mouvement relatif est produit entre la caméra et le système d'éclairage d'une part, et la pièce à observer d'autre part, de façon à pouvoir inspecter l'ensemble d'une face plane, même avec une caméra linéaire ou une caméra matricielle dont le champ de vision est réduit. Les défauts de structure sont pour l'essentiel décelés à partir de variations de luminance des signaux vidéo délivrés par une caméra d'observation. De tels systèmes d'observation sont toutefois mal adaptés à la reconnaissance de particularités géométriques de pièces, par exemple des particularités géométriques constituées par des parties chanfreinées ou flaches sur des rives d'une pièce de bois adjacentes à une face principale observée.

On connaît par ailleurs des systèmes d'observation d'objets en défilement qui comprennent des sources laser projetant une lumière cohérente sur l'objet à observer et des capteurs photo-électriques recevant la lumière cohérente des sources laser réfléchie par l'objet de façon à permettre une détection de défauts à partir des variations d'amplitude de la lumière réfléchie. De tels systèmes d'observation d'objets ne sont toutefois pas adaptés à une inspection complète d'un objet permettant de déceler à la fois des défauts ou des particularités géométriques et des défauts de coloration.

La présente invention a pour but de remédier aux inconvénients de l'art antérieur et notamment de permettre de façon simple et efficace de détecter de façon optique des particularités géométriques affectant des faces secondaires d'un objet adjacentes à une face principale de cet objet inspectée par une caméra vidéo.

L'invention vise de façon plus particulière à permettre de détecter simultanément par un procédé optique à la fois des défauts ou des particularités géométriques affectant deux faces secondaires d'un objet adjacentes à une face principale, et des défauts de structure et des défauts de coloration présents sur ladite face principale de la pièce.

Ces buts sont atteints grâce à un procédé de reconnaissance de particularités géométriques de pièces parallélépipédiques de section polygonale comportant au moins une face secondaire adjacente à une face principale, l'angle maximum défini entre la face principale considérée et ladite face secondaire adjacente étant compris entre 0° et 90°, caractérisé en ce qu'il comprend les étapes suivantes:

a) disposer une source de lumière monochromatique projetant un faisceau plan de rayonnement collimaté et monochromatique perpendiculairement à l'axe longitudinal de la pièce de manière à éclairer de face ladite face secondaire sans éclairer la surface plane de la face principale,

b) disposer une caméra vidéo linéaire couleur en regard de la face principale considérée, de telle sorte que son axe soit perpendiculaire à ladite face principale et qu'elle observe une ligne perpendiculaire à l'axe longitudinal de la pièce, dans le plan du faisceau de rayonnement collimaté et monochromatique, de manière à observer à la fois ladite face principale et la projection de ladite face secondaire adjacente sur la face principale,

c) acquérir simultanément les trois signaux couleur R, V, B issus de la caméra, chaque signal représentant l'amplitude de l'une des trois composantes de base R, V, B en chaque point analysé suivant la ligne d'observation,

d) traiter en temps réel les signaux R, V, B pour fournir une succession de pixels adjacents et associés selon l'appartenance de leurs composantes R, V, B à des domaines particuliers de l'espace R, V, B identifiés par des labels,

e) produire un déplacement longitudinal relatif de la pièce à analyser par rapport à la caméra et à la source de lumière monochromatique pour observer l'ensemble de la face principale et la projection de la face secondaire adjacente sur ladite face principale, et

f) reconnaître les particularités géométriques de ladite face secondaire à partir de l'identification des pixels correspondant à un domaine particulier de l'espace R, V, B identifié par un label correspondant à l'éclairage monochromatique de ladite source de lumière monochromatique.

De façon particulière, selon l'invention, on dispose en outre une source d'éclairage diffus à lumière polychromatique entre la caméra et la face principale de la pièce pour éclairer de façon diffuse la ligne de la face principale observée par la caméra, et on procède à une identification simultanée de défauts de structure ou de coloration de la face principale et de particularités géométriques de la face secondaire adjacente, dans l'image captée par la caméra et pré-traitée en temps réel à l'aide d'une opération de classification de pixels par couleur, chaque pixel de l'image captée étant associé à un sous-ensemble spécifique identifié par un label, ce qui permet de caractériser directement l'appartenance du pixel à une entité spécifique de l'image indépendamment de toute autre caractéristique de dimension ou de forme de la région d'image à laquelle appartient ledit pixel, et permet ainsi de détecter et discriminer d'une part lesdites particularités géométriques de ladite face secondaire et d'autre part des défauts de structure ou de coloration de la face principale représentés par des pixels correspondant à des domaines particuliers de l'espace R, V, B identifiés par des labels ne correspondant pas audit éclairage monochromatique de la source de lumière monochromatique.

Selon une caractéristique avantageuse, lors de l'acquisition simultanée des trois signaux couleur R, V, B issus de la caméra linéaire, le traitement en temps réel des signaux R, V, B comprend un changement d'espace couleur réalisé électroniquement pour définir la couleur des points dans un nouvel espace tridimensionnel favorisant la discrimination des couleurs.

Selon une autre caractéristique préférentielle, lors de l'acquisition simultanée des trois signaux couleur R, V, B issus de la caméra linéaire, on corrige électroniquement en temps réel l'intensité des composantes R, V, B reçue par chaque pixel afin de compenser d'éventuelles hétérogénéités d'éclairage.

L'opération de classification des pixels en sous-ensembles spécifiques munis d'un label selon la couleur s'effectue en une première phase préalable d'apprentissage des couleurs caractéristiques de chaque singularité à identifier dans l'image et à une deuxième phase de classification proprement dite en temps réel au cours de laquelle chaque pixel n'est plus caractérisé par un triplet de coordonnées dans un espace couleur, mais par le seul label auquel appartient la couleur correspondant à ce triplet de coordonnées.

Selon un mode particulier de réalisation, l'image couleur labellisée est soumise à une opération de filtrage, puis de segmentation de régions, afin de définir les attributs dimensionnels et géométriques de chaque singularité de structure ou de coloration et de chaque particularité géométrique présentes dans l'image captée par la caméra linéaire.

De façon paticulière, le procédé selon l'invention peut comprendre une opération d'identification des défauts qui s'effectue en une première phase préalable d'apprentissage des défauts types et de détermination des paramètres les plus discriminants permettant d'identifier chaque type de défaut, et à une deuxième phase de reconnaissance des défauts en temps réel, au cours de laquelle il est déterminé un ensemble de paramètres décrivant chaque défaut extrait, et selon la valeur prise par chacun de ces paramètres, il est calculé la probabilité d'occurrence de chaque défaut préalablement appris et les types de défaut associés aux plus fortes probabilités d'occurrence sont alors considérés comme reconnus.

Le procédé selon l'invention peut encore être caractérisé en ce que l'on procède simultanément à la reconnaissance de particularités géométriques de plusieurs faces secondaires adjacentes à des faces principales et à une identification simultanée de défauts de structure ou de coloration desdites faces principales en disposant autant de sources de lumière monochromatique qu'il y a de faces secondaires et autant de caméras vidéo linéaires couleur qu'il y a de faces principales, et en ce que lors de l'acquisition des signaux couleur R, V, B issus de chaque caméra linéaire, on constitue des lignes d'image comprenant la juxtaposition de pixels issus des différentes caméras linéaires.

L'invention s'applique également à un dispositif de reconnaissance de particularités géométriques de pièces parallélépipédiques de section polygonale comportant au moins une face secondaire adjacente à une face principale, l'angle maximum défini entre la face principale considérée et ladite face secondaire adjacente étant compris entre 0° et 90°, caractérisé en ce qu'il comprend au moins

a) une source de lumière monochromatique projetant un faisceau plan de rayonnement collimaté et monochromatique perpendiculairement à l'axe longitudinal de la pièce de manière à éclairer de face ladite face secondaire sans éclairer la surface plane de la face principale,

b) une caméra vidéo linéaire couleur située en regard de la face principale considérée, de telle sorte que son axe soit perpendiculaire à ladite face principale et qu'elle observe une ligne perpendiculaire à l'axe longitudinal de la pièce, dans le plan du faisceau de rayonnement collimaté et monochromatique,

c) des moyens de support et d'entraînement pour provoquer un mouvement relatif entre d'une part la caméra et la source de lumière monochromatique et d'autre part la pièce dans le sens de l'axe longitudinal de celle-ci de telle manière que des portions successives de la face principale et de la face secondaire de la pièce sont respectivement observées et éclairées par la caméra et la source de lumière monochromatique,

d) des moyens électroniques d'acquisition et de traitement en temps réel

des signaux couleur R, V, B délivrés par la caméra linéaire, pour fournir une succession de pixels adjacents et associés selon l'appartenance de leurs composantes R, V, B à des domaines particuliers de l'espace R, V, B identifiés par des labels, et permettre la reconnaissance des particularités géométriques de ladite face secondaire à partir de l'identification des pixels correspondant à un domaine particulier de l'espace R, V, B identifié par un label correspondant à l'éclairage monochromatique de ladite source de lumière monochromatique.

Avantageusement, le dispositif comprend en outre une source d'éclairage diffus à lumière polychromatique interposée entre la caméra et la face principale de la pièce pour éclairer de façon diffuse la ligne de la face principale observée par la caméra et les moyens électroniques d'acquisition et de traitement en temps réel des signaux couleur R, V, B comprennent des moyens de discrimination pour détecter et discriminer d'une part lesdites particularités géométriques de ladite face secondaire et d'autre part des défauts de structure ou de coloration de la face principale.

Selon un mode particulier de réalisation, la source d'éclairage diffus à lumière polychromatique comprend des première et deuxième sources lumineuses disposées transversalement par rapport à la pièce à observer, de part et d'autre du plan contenant les axes de la caméra et de la source de lumière monochromatique, des réflecteurs destinés à orienter la lumière polychromatique émise par lesdites première et deuxième sources lumineuses vers la ligne transversale observée par la caméra et un carter de support des sources lumineuses et des réflecteurs, lequel carter comprend une ouverture transversale en forme de fente permettant à la caméra d'observer la ligne constituée par l'intersection entre les faisceaux de lumière polychromatique et la face principale de la pièce.

La source de lumière monochromatique peut comprendre un générateur laser ou une source de lumière polychromatique à laquelle est adjoint un filtre coloré.

Le dispositif selon l'invention est avantageusement appliqué à des pièces parallélépipédiques de section rectangulaire présentant deux faces principales opposées et deux faces secondaires ou rives.

Dans ce cas, le dispositif comprend selon un premier plan perpendiculaire à l'axe de la pièce des première et deuxième caméras vidéo linéaires couleur disposées en regard respectivement des première et deuxième faces principales opposées de la pièce, des première et deuxième sources de lumière monochromatique situées en regard respectivement des première et deuxième faces secondaires opposées, des première et deuxième sources de lumière polychromatique interposées entre les première et deuxième caméras et les première et deuxième faces principales opposées de la pièce et des moyens électroniques d'acquisition et de traitement en temps réel des signaux couleur R, V, B délivrés par les deux caméras linéaires.

Avantageusement, pour permettre un examen complet de l'ensemble des quatre faces de la pièce observée, le dispositif comprend en outre selon un deuxième plan perpendiculaire à l'axe de la pièce et décalés longitudinalement par rapport audit premier plan perpendiculaire à l'axe de la pièce, des troisième et quatrième caméras vidéo linéaire couleur disposées en regard respectivement des première et deuxième faces secondaires opposées de la pièce, des troisième et quatrième sources de lumière monochromatique situées en regard respectivement des première et deuxième faces principales opposées, des troisième et quatrième sources de lumière polychromatique interposées entre les troisième et quatrième caméras et les première et deuxième faces secondaires opposées et des moyens électroniques d'acquisition et de traitement en temps réel des signaux couleur R, V, B délivrés par l'ensemble des quatre caméras linéaires.

L'invention peut s'appliquer à des pièces parallélépipédiques de différentes natures en matériau artificiel ou naturel. Toutefois, une application particulièrement utile est constituée par l'application à des pièces de bois dans lesquelles on souhaite détecter des défauts de structure tels que des noeuds ou fentes, ainsi que des défauts de coloration, tout en détectant simultanément des défauts ou des particularités géométriques, telles que des flaches ou chanfreins, affectant les rives adjacentes à une face principale observée.

L'invention qui permet une analyse de pièces de section polygonale sur une ou plusieurs faces durant un déplacement longitudinal des pièces peut trouver des applications dans:

- le tronçonnage automatique et optimisé d'avivés (pièces de bois de section rectangulaire),

- le délignage d'avivés ou de plateaux flacheux (pièces de bois de section approximativement trapézoïdale),

- le triage qualitatif de pièces en sortie de raboteuse (pièces de bois usinées à deux faces parallèles intégrant des profils).

D'autres caractéristiques et avantages de l'invention ressortiront de la description suivante de modes particuliers de réalisation, donnés à titre d'exemples non limitatifs en référence aux dessins annexés, sur lesquels :

- la figure 1 est une vue en bout schématique montrant un premier exemple de dispositif simplifié selon l'invention permettant de détecter des particularités géométriques d'une pièce parallélépipédique,

- la figure 2 est une vue en bout similaire à celle de la figure 1, mais montrant un dispositif plus complet d'observation optique selon l'invention, avec un dispositif d'éclairage diffus et deux sources de rayonnement laser,

- les figures 3 et 4 sont des vues du dispositif de la figure 2, respectivement de côté et de dessus,

- la figure 5 est une vue en bout similaire à celle de la figure 2, mais montrant un dispositif d'observation optique encore plus complet permettant l'observation simultanée et complète de quatre faces d'une pièce parallélépipédique,

- la figure 6 est une vue en perspective du dispositif de la figues 5 montrant la forme des faisceaux lumineux de lumière monochromatique et le champ d'observation des caméras, mais dans laquelle les dispositifs d'éclairage diffus ont été éliminés pour des besoins de clarté,

- les figures 7A, 7B, 7C et 7D sont des vues des quatre faces de la pièce de la figure 6 examinées simultanément par le dispositif de la figure 6,

- la figure 8 est une vue de côté d'un exemple de dispositif d'éclairage diffus utilisable dans le cadre de la présente invention,

- la figure 9 est une vue en bout du dispositif d'éclairage de la figure 8, partiellement en coupe selon la ligne IX-IX de cette figure 8,

- la figure 10 est une vue de dessus du dispositif d'éclairage des figures 8 et 9,

- la figure 10A est une vue en coupe transversale d'un réflecteur incorporé dans le dispositif d'éclairage diffus des figures 8 à 10,

- la figure 11 est un organigrame montrant les différentes étapes de traitement des signaux émis par des caméras linéaires couleur du dispositif selon l'invention, et

- la figure 12 est un schéma-bloc montrant différents blocs fonctionnels des circuits électroniques de traitement des signaux émis par des caméras linéaires couleur du dispositif selon l'invention.

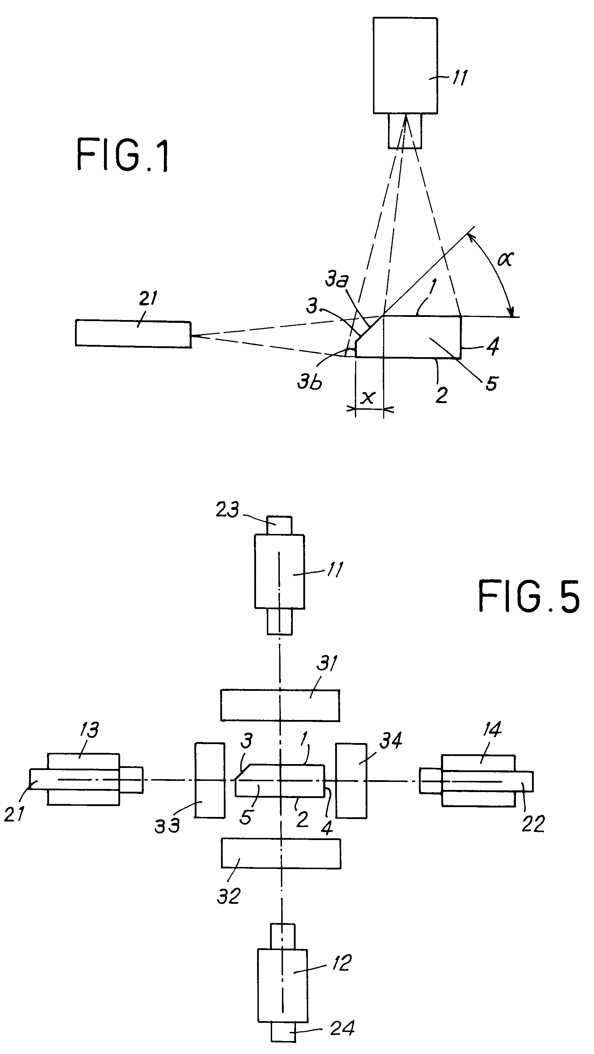

On voit sur la figure 1 les deux éléments essentiels d'un dispositif d'observation selon l'invention adapté à l'inspection automatique d'une pièce parallélépipédique 5 à section polygonale comprenant une face principale supérieure 1, une face principale inférieure 2 et des faces secondaires latérales ou rives 3, 4.

La pièce 5, qui peut être une pièce de bois comprend une face latérale 3 dont une partie 3a est chanfreinée et présente un angle aigu a avec la face principale supérieure 1 et une autre partie 3b est sensiblement perpendiculaire à cette face supérieure 1.

Le dispositif d'observation comprend une caméra vidéo linéaire couleur 11, qui est située au-dessus de la face principale 1, et dont l'axe est perpendiculaire à cette face principale 1. La caméra 11 observe une ligne perpendiculaire à l'axe longitudinal de la pièce 5 et présente un champ suffisant pour pouvoir observer à la fois la face principale 1 et la projection x de la face secondaire 3 sur la face principale 1.

Une source 21 de lumière monochromatique est en outre disposée latéralement en regard de la face latérale 3, de manière à projeter un faisceau plan de rayonnement collimaté et monochromatique perpendiculairement à l'axe longitudinal de la pièce 5 de manière à éclairer de face la face latérale 3 sans éclairer la surface plane de la face principale 1. Le faisceau plan issu de la source 21 se trouve dans le plan du champ d'observation du capteur linéaire de la caméra 11.

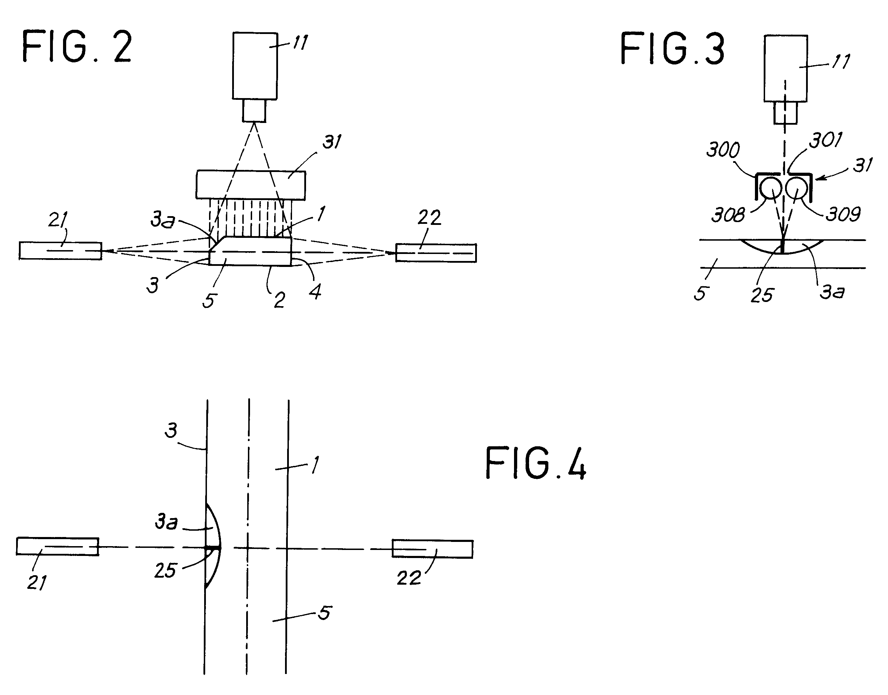

La face plane principale 1 de la pièce 5 est par ailleurs éclairée par un éclairage diffus de lumière polychromatique qui peut résulter de l'éclairage ambiant mais provient de préférence d'une source spécifique incorporée à l'ensemble du dispositif d'observation comme cela sera décrit plus loin en référence aux figures 2, 3, 5, 8 à 10.

Le faisceau de rayonnement monochromatique peut provenir d'une source laser ou peut être obtenu à partir d'une source de lumière polychromatique à laquelle est adjoint un filtre coloré.

La source 21 de lumière monochromatique comprend en outre un dispositif optique (par exemple du type lentille semi-cylindrique) ou mécanique (par exemple de type prisme ou miroir tournant) pour engendrer un faisceau lumineux plan perpendiculaire à l'axe de la pièce 5 et se projetant sur la face latérale 3 de celle-ci dans le plan contenant l'axe optique de la caméra 11 et la ligne observée sur la surface analysée 3.

La caméra vidéo linéaire couleur 11 peut être choisie parmi différents types possibles.

A titre d'exemple, selon un premier type possible, on utilise une caméra linéaire mono-CCD dans laquelle le capteur est constitué d'une juxtaposition d'éléments sensibles à l'une des trois couleurs de base rouge (R), vert (V) et bleu (B).

L'agencement des éléments suivant la longueur du capteur est alors organisé suivant plusieurs séquences R V B : ...RVB, RVB, RVB, ... Le nombre de points d'image identifiables par ce capteur est égal au nombre de triplets RVB le constituant.

Selon un deuxième type possible, on utilise une caméra linéaire tri-CCD réalisée à partir de trois capteurs linéaires.

Dans ce cas, chaque capteur est formé par la juxtaposition d'éléments sensibles à l'une des trois couleurs de base. La caméra intègre donc un premier capteur linéaire sensible à la couleur rouge, un deuxième capteur linéaire sensible à la couleur bleue et un troisième capteur linéaire sensible à la couleur verte.

Ces trois capteurs sont physiquement parallèles entre eux et positionnés dans un plan parallèle à la surface observée. Dans ce cas, les trois capteurs physiquement écartés d'une certaine distance, n'observent pas simultanément la même ligne d'image. Une remise en phase temporelle doit alors être réalisée par le dispositif électronique d'acquisition d'image.

Selon un troisième type possible, on utilise une caméra linéaire tri CCD constituée de trois capteurs similaires à ceux du modèle précédemment décrit mais positionnés dans un plan perpendiculaire à celui de la surface observée. Dans ce plan les capteurs sont agencés selon une forme en "U" où la base est parallèle à la surface observée et les deux côtés sont perpendiculaires. Dans ce cas, un dispositif optique (de type prisme) illumine simultanément les trois capteurs qui observent donc la même ligne d'image.

Quel que soit le type de caméra utilisé dans cette application, la caméra observe la réflexion diffuse issue de l'éclairage à lumière polychromatique ou de l'éclairage à lumière monochromatique.

Lors de l'utilisation d'une caméra du deuxième type, la caméra est réglée de telle sorte que l'éclairage collimaté à lumière monochromatique soit aligné sur le capteur le plus sensible à la longueur d'onde de la lumière issue dudit éclairage.

Le dispositif selon l'invention comprend des moyens d'acquisition simultanée des trois signaux couleur R V B issus de la caméra 11, chaque signal représentant l'amplitude de l'une des trois composantes de base R V B en chaque point analysé suivant la ligne d'observation.

Les signaux couleur R V B sont traités en temps réel pour fournir une succession de pixels adjacents et associés selon l'appartenance de leurs composantes R V B à des domaines particuliers de l'espace R, V, B identifiés par des labels.

Un déplacement longitudinal relatif est produit entre la pièce 5 à analyser et le dispositif d'observation comprenant la caméra 11 et la source 21 de lumière monochromatique. Généralement, il est plus commode d'installer la caméra 11 et la source 21 à poste fixe et de provoquer un défilement de la pièce 5 à analyser, mais on peut aussi observer une pièce 5 fixe à l'aide d'un dispositif d'observation comprenant une caméra 11 et une source 21 de lumière monochromatique qui présentent des positions relatives déterminées et sont montées sur un support de type chariot pouvant se déplacer simultanément, par exemple le long de rails s'étendant parallèlement à la direction longitudinale de la pièce 5. Lors du déplacement longitudinal relatif entre la pièce 5 et le dispositif d'observation, il est ainsi possible de réaliser une inspection de l'ensemble de la face principale et de la projection de la face secondaire 3 dans le plan de la face principale 1.

Les particularités géométriques de la face latérale 3, telles que la présence de chanfreins 3a, sont observées à partir de l'identification des pixels correspondant à un domaine particulier de l'espace R V B identifié par un label correspondant à l'éclairage monochromatique de la source 21 de lumière monochromatique.

Dans le cas où l'on souhaite observer simultanément les deux faces latérales 3, 4 adjacentes à une face principale 1 de la pièce 5 à l'aide d'une seule caméra 11 dont le champ de vision couvre à la fois la face principale 1 et les projections des faces latérales 3, 4 dans le plan de la face principale 1, il suffit de disposer une deuxième source de lumière monochromatique 22 face à la deuxième face 4, comme représenté sur les figures 2 et 4. Les sources 21 et 22 peuvent être identiques et projettent toutes deux un faisceau plan de rayonnement monochromatique collimaté perpendiculairement à l'axe longitudinal de la pièce 5, les faisceaux plans se situant dans le plan d'observation de la caméra 11 et n'éclairant pas la surface plane de la face principale 1.

Sur les figures 3 et 4, on voit la trace 25 du faisceau de lumière monochromatique de la source 21 qui permet de déceler la présence d'une partie chanfreinée 3a au niveau de la face latérale 3 de la pièce 5.

Les figures 2 et 3 montrent en outre une source 31 d'éclairage diffus à lumière polychromatique interposé entre la caméra 11 et la face principale 1 pour éclairer de façon diffuse la ligne de la face principale 1 observée par la caméra 11.

La source 31 d'éclairage diffus comprend un carter 300 dans lequel est ménagée une ouverture 301 en forme de fente, disposée transversalement par rapport à la pièce 5 pour permettre à la caméra 11 d'observer la ligne constituée par l'intersection entre les faisceaux de lumière polychromatique et la face principale 1 de la pièce 5, ainsi que les lignes 25 de lumière monochromatique déterminant les particularités géométriques des faces latérales 3, 4 adjacentes à la face principale 1.

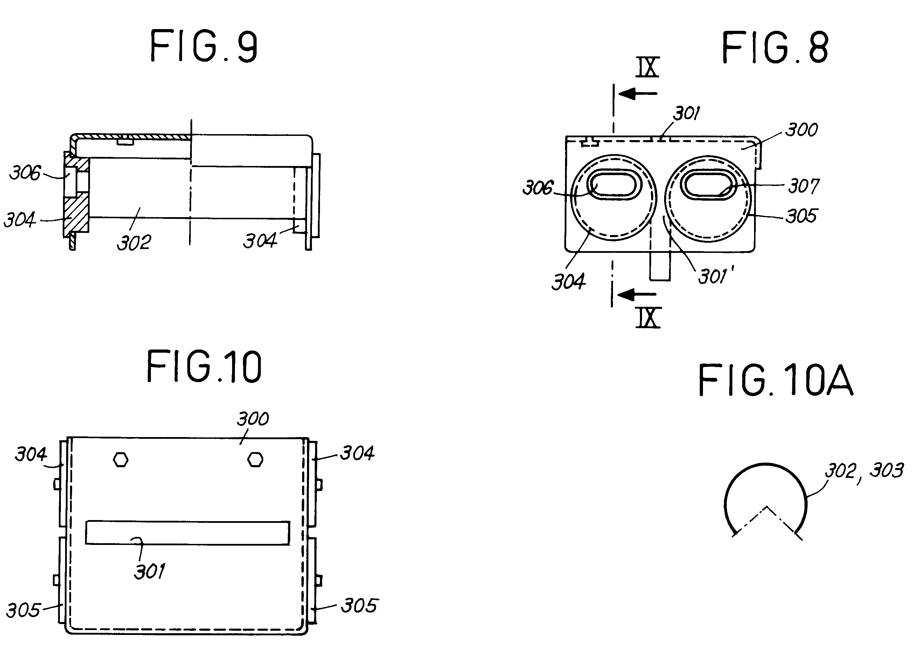

Le carter 300 supporte des première et deuxième sources lumineuses 308, 309 qui sont disposées transversalement par rapport à la pièce 5 à observer de part et d'autre du plan contenant les axes de la caméra 11 et des sources de lumière monochromatique 21, 22. Les sources lumineuses 308, 309 peuvent être constituées par deux lampes alimentées en courant continu ou en courant alternatif délivré par des ballasts haute fréquence qui minimisent les éventuelles variations de lumière. Le carter 300 supporte également des réflecteurs 302, 303 qui intègrent chacun une des lampes 308, 309 et orientent la lumière émise par celles-ci sur la ligne observée par la caméra 11. Un exemple de réflecteurs cylindriques 302, 303 est représenté en coupe transversale sur la figure 10A, avec une ouverture inférieure s'étendant selon un angle de l'ordre de 100°.

Les figures 8, 9 et 10 montrent la forme d'un exemple particulier de carter 300 de source 31 d'éclairage diffus. Ces figures 8 à 10 montrent les éléments d'extrémité 304, 305 en forme de disques munis d'ouvertures oblongues 306, 307 pour recevoir les extrémités des lampes 308, 309, non représentées sur les figures 8 à 10. Les disques 304, 305, eux-mêmes fixés sur le carter 300 peuvent également servir d'éléments de montage pour les réflecteurs 302, 303.

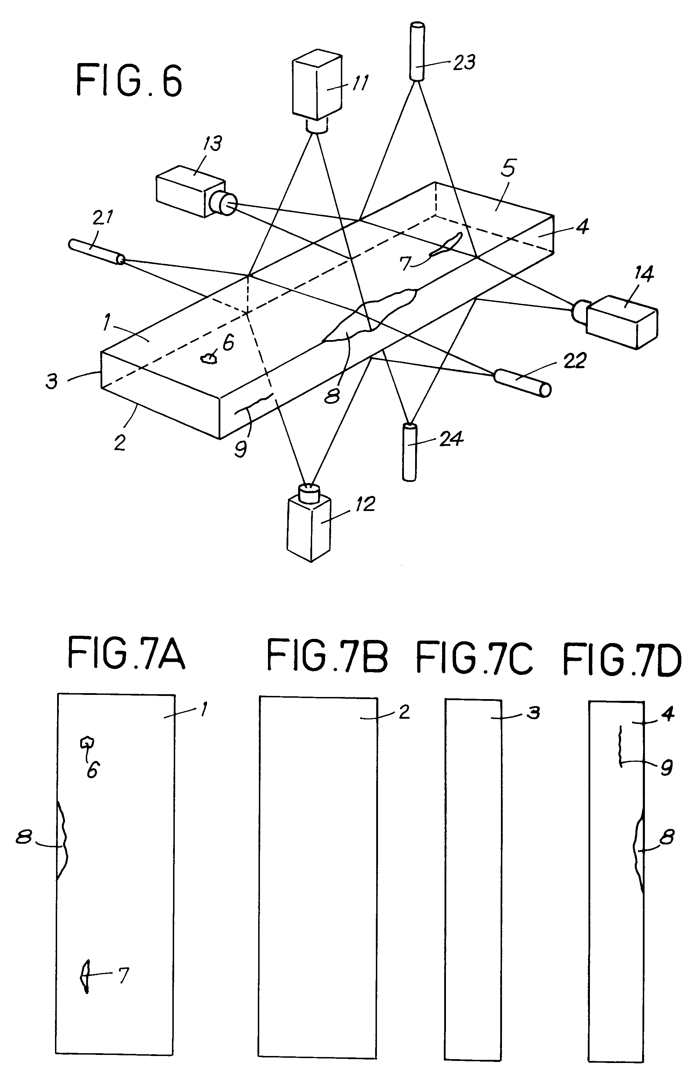

Les figures 5 et 6 montrent un exemple de dispositif selon l'invention permettant d'analyser de façon complète les quatre faces d'une pièce parallélépipédique 5 à section rectangulaire ou trapézoïdale. Sur la figure 6, qui est une vue en perspective, les dispositifs tels que 31 d'éclairage diffus n'ont pas été représentés par souci de clarté.

Dans le dispositif des figures 5 et 6, une caméra linéaire couleur supplémentaire 12 est disposée dans le même plan que la caméra 11, de façon symétrique par rapport à la pièce 5, de façon à observer la face plane principale inférieure 2 de la pièce 5.

Comme on peut le voir sur les figures 5 et 6, la mise en oeuvre, dans un même plan transversal, de deux caméras linéaires 11, 12, de deux sources 21, 22 de lumière monochromatique, ainsi que de deux sources de lumière diffuse polychromatique 31, 32 dont l'objet est de faire ressortir les défauts de structure et de coloration permet à la fois d'opérer une reconnaissance des particularités géométriques des faces latérales 3, 4 et de détecter les défauts de structure et de coloration des faces principales 1, 2 de la pièce 5.

Dans les cas où il est souhaitable de pouvoir détecter sur chacune des faces 1 à 4 de la pièce 5 à la fois les particularités géométriques et les défauts de structure et de coloration, l'équipement mentionné au paragraphe précédent est doublé dans un plan transversal décalé par rapport au plan transversal défini par les éléments 11, 12 et 21, 22.

L'ensemble supplémentaire de deux caméras 13, 14, deux éclairages polychromatique diffus 33, 34 et deux éclairages monochromatiques collimatés 23, 24 subit une rotation de 90° autour de l'axe de symétrie du dispositif par rapport au premier ensemble de deux caméras 11, 12, de deux éclairages polychromatiques diffus 31, 32 et deux éclairages monochromatiques collimatés 21, 22, (voir figures 5 et 6). Le décalage longitudinal entre les plans transversaux définis par ces deux ensembles d'observation peut être de quelques centimètres, par exemple de l'ordre de 20 cm, afin d'éviter que les éclairage collimatés de ces deux ensembles d'observation ne se perturbent mutuellement.

On a représenté sur la figure 6 et les figures 7A à 7D des exemples de défauts ou particularités géométriques pouvant être observés à l'aide du dispositif selon l'invention, tel qu'il a été décrit en référence aux figures 5 et 6.

On voit ainsi sur la face plane supérieure 1 de la pièce de bois 5 la trace d'un noeud 6, d'une fente 7 et d'un chanfrein ou flache 8 (figure 7A). La face plane inférieure 2 est en revanche exempte de défauts (figure 7B), de même que la face latérale 3 (figure 7C). La figure 7D montre à la fois la trace du chanfrein 8 déjà repéré sur la figure 7A et une fente 9.

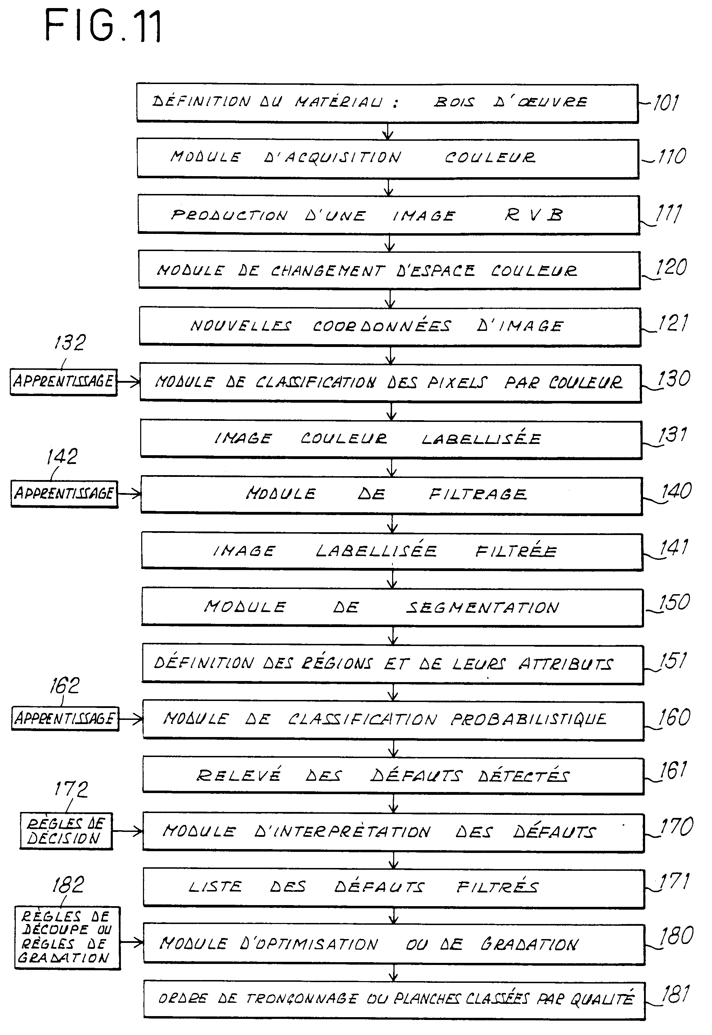

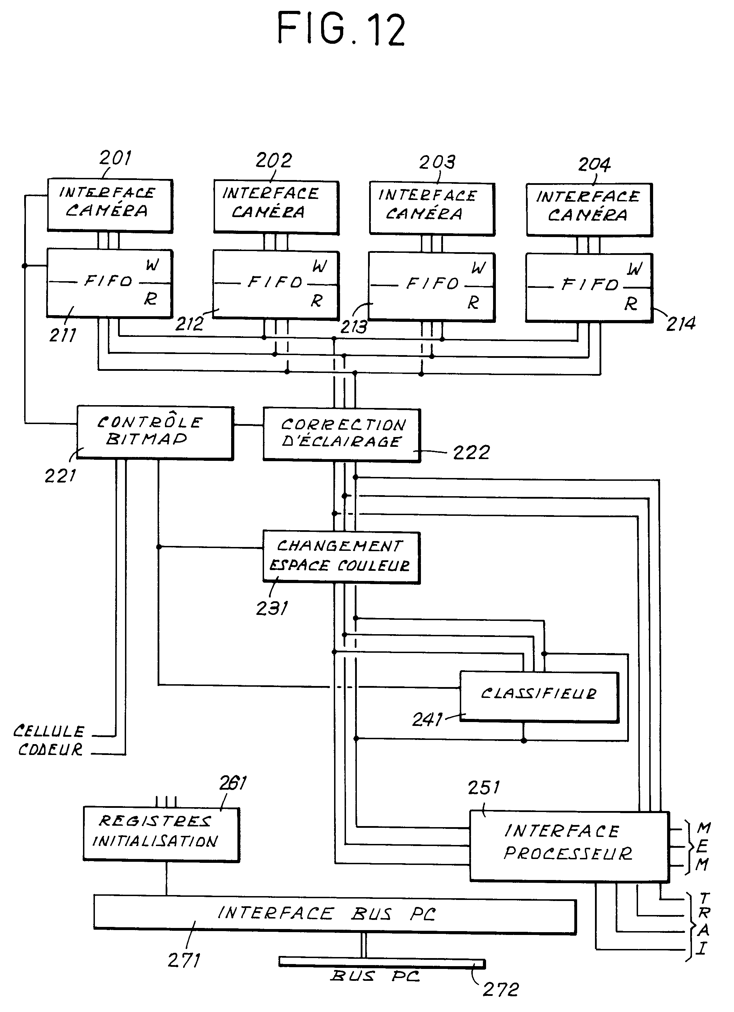

On décrira maintenant en référence aux figures 11 et 12 les dispositifs de traitement des signaux couleur R V B délivrés par une ou plusieurs caméras linéaires couleur 11, 12, 13, 14.

La figure 11 montre sous forme d'organigramme les différentes étapes mises en oeuvre dans des dispositifs de traitement des signaux couleur R V B issus des caméras 11 à 14.

Dans une étape préliminaire 101, on définit les caractéristiques particulières du matériau de la pièce qui va être analysée. Par exemple, on indique que la pièce est constituée par du bois d'oeuvre. Ces informations permettent de prendre en compte les résultats de procédures d'apprentissage 142, 162, ou des séries de règles 172, 182, établies au préalable pour le type de matériau considéré.

Un module d'acquisition couleur 110 a pour fonction d'acquérir simultanément les trois signaux R, V, B issus des caméras 11 à 14. Chaque signal représente l'amplitude d'une des trois composantes de base R, V, B en chaque point analysé suivant une ligne d'observation d'une caméra sur la pièce 5.

Dans le module d'acquisition couleur 110, on procède à la formation de lignes d'image constituées par la juxtaposition de pixels issus de une, deux, trois ou quatre caméras 11 à 14. Il est possible de sélectionner sur chaque caméra une zone quelconque de pixels consécutifs, puis de former une seule ligne d'image par concaténation des zones issues de chaque caméra.

Ceci est effectué à l'aide de circuits FIFO (premier entré, premier sorti) 211 à 214 de gestion de files d'attente qui reçoivent les signaux couleur R, V, B des interfaces 201 à 204 associées aux différentes caméras linéaires couleur 11 à 14 (figure 12).

Un circuit 222 de correction d'éclairage, qui fonctionne en temps réel en liaison avec un circuit 221 de contrôle de synchronisation, fait également partie du module d'acquisition couleur 110 à partir duquel il est produit une image couleur R V B (étape 111).

Dans le circuit de correction d'éclairage 222, l'intensité des composantes R, V, B reçue par chaque pixel est corrigée électroniquement en temps réel afin de compenser d'éventuelles hétérogénéités d'éclairage.

La fonction de correction est du type

$$I_c = A \times I_e + B$$

où

$I_c$ = Intensité corrigée

$I_e$ = Intensité émise

A et B = coefficients de correction

Par ailleurs, lors de l'acquisition des signaux couleur, la couleur de chaque pixel est généralement caractérisée par un triplet de coordonnées définies dans un espace tridimensionnel du type (R, V, B) = Rouge, Vert, Bleu.

Toutefois, cet espace tridimensionnel n'est pas nécessairement le plus intéressant et il peut être préférable de caractériser la couleur de chaque pixel par un triplet de coordonnées défini dans un autre espace tridimensionnel, tel que par exemple du type (T, L, S) = Teinte, Luminance, Saturation.

Dans le cas où la caméra linéaire couleur utilisée ne délivre pas directement les coordonnées colorimétriques dans l'espace couleur le plus intéressant, il est procédé automatiquement de façon électronique à un changement d'espace dans un module 120 de changement d'espace couleur (correspondant aux circuits 231 de la figure 12).

Le triplet de coordonnées correspondant aux nouvelles coordonnées d'image fournies au niveau de l'étape 121, s'exprime alors sous une forme du type :

(f(R, V, B); g (R, V, B); h (R, V, B))

où f, g et h sont des formes linéaires de R, V, B permettant de définir la couleur des points dans un autre espace tridimensionnel favorisant la discrimination des couleurs.

Les pixels caractérisés par des triplets de coordonnées dans l'espace couleur choisi dans le module 120 sont traités dans un module 130 de classification des pixels par couleur (correspondant aux circuits 241 de la figure 12) afin de foumir à l'étape 131 une image couleur labellisée.

A l'étape 121 d'entrée dans le module de classification des pixels par couleur, l'ensemble des valeurs possibles de l'espace est explicité sous forme de triplets (C1, C2, C3). A chaque triplet d'adresse est associé une forme ou label choisi parmi l'ensemble des labels disponibles:

| Adresses | Données |
|---|---|
| (C1.1, C2.1, C3.1) | ......> Li |
| (C1.1, C2.1, C3.2) | .....> Lj |
| (C1.1, C2.1, C3.3) | .....> Lk |
| ... | |
| (C1.n, C2.n, C3.n) | .....> Ln |

où :

C1i = f (R, V, B)

C2i = g (R, V, B)

C3i = h (R, V, B)

Physiquement, chaque label (Li, Lj ....) regroupe l'ensemble des couleurs caractérisant une entité particulière.

Le simple fait d'associer un pixel de l'image à un sous-ensemble spécifique (label) permet de caractériser directement son appartenance à une entité spécifique de l'image indépendamment de toute autre caractéristique de dimension, de forme .... de la région d'image à laquelle appartient le dit pixel.

Cette opération de classification des pixels s'effectue en deux phases :

a) Apprentissage des couleurs caractéristiques de chaque singularité à identifier dans l'image, (module 132)

b) Classification proprement dite où chaque pixel n'est plus caractérisé par un triplet de coordonnées (C1, C2, C3) mais par son seul label (module 130)

La phase d'apprentissage 132 est réalisée par un opérateur humain qui localise dans une image préalablement enregistrée chaque zone correspondant à une singularité spécifique.

L'opérateur communique également au système informatique le type de la singularité localisée.

Cette opération de localisation et d'identification de singularités est répétée plusieurs fois afin que le système informatique dispose d'une diversité de données suffisante pour sélectionner le mieux possible les couleurs à regrouper dans un même label.

La phase de classification proprement dite est ensuite réalisée électroniquement, et chaque pixel est alors caractérisé par son label (étape 131).

L'image couleur labellisée présente à l'étape 131, et pour laquelle l'appartenance d'un pixel à un sous-ensemble suffit à définir le type de singularité à laquelle il est intégré, est traitée dans un module de filtrage 140 associé à une procédure préalable d'apprentissage dans une étape 142.

Le module de filtrage 140 vise à permettre d'effectuer une distinction entre les singularités de structure ou de coloration et les particularités géométriques.

Dès lors que l'appartenance d'un pixel à un sous-ensemble suffit à définir le type de singularité à laquelle il est intégré, chaque singularité de structure ou de coloration mise en évidence par l'éclairage diffus 31 à 34 est directement identifiable par sa couleur propre.

En revanche, les particularités géométriques affectant par exemple les faces latérales secondaires 3, 4 ou rives de la surface plane principale observée 1 ou 2 de la pièce 5 sont directement illuminées par l'éclairage 21 et 22 à lumière monochromatique. Ces particularités ne sont alors plus caractérisables à partir de leur couleur propre mais à partir de la couleur de la lumière monochromatique projetée sur l'ensemble de leur surface.

La simple identification par filtrage du label correspondant à la couleur de l'éclairage, permet donc de caractériser une particularité géométrique affectant une rive 3, 4 de la surface analysée 1 ou 2.

L'image labellisée filtrée obtenue à l'étape 141 après passage dans le module de filtrage 140 est ensuite traitée informatiquement dans un module 150 de segmentation de régions dont la fonction est de définir les attributs dimensionnels et géométriques de chaque entité présente dans l'image.

Chacune des singularités de structure ou de coloration et chaque particularité géométrique est ainsi caractérisée par son type, sa forme, sa dimension, sa localisation (X, Y).

Une fois disponible à l'étape 151 la définition des régions et de leurs attributs, il est procédé à une identification des défauts dans un module 160 de classification probabiliste.

Dans ce module 160, les défauts sont identifiés à l'aide d'une procédure statistique organisée en deux étapes:

Dans une première étape 162 d'apprentissage, les images de défauts types sont stockées en mémoire du système d'analyse d'image. Un opérateur humain repère chaque défaut présent dans l'image et le nomme.

Le système détermine alors quels sont les paramètres les plus discriminants permettant d'identifier chaque type de défaut.

Dans une deuxième étape, on procède à une reconnaissance des défauts en exploitation continue, le système de vision détermine un ensemble de paramètres décrivant chaque défaut extrait (surface, périmètre...).

Selon la valeur prise par chacun des paramètres, le système calcule ensuite la probabilité d'occurrence de chaque défaut préalablement appris.

Le type de défaut associé à la plus forte probabilité d'occurrence est alors considéré comme reconnu.

Une fois disponible à l'étape 161 le relevé des défauts détectés, on procède dans un module 170 d'interprétation des défauts, à un filtrage en fonction de règles de décision préalablement déterminées dans une étape 172 afin de ne retenir que des défauts significatifs pour l'étude de la pièce analysée 5.

On obtient ainsi à l'étape 171 une liste des défauts filtrés. Ces informations peuvent le cas échéant être utilisées dans le cadre d'un module 180 d'optimisation ou de gradation qui fonctionne avec des règles de découpe ou des règles de gradation préalablement introduites dans une étape 182.

Le système de vision ayant élaboré aux étapes 170, 171 la liste des défauts présents sur les pièces analysées (numéro de face, type de défaut, rectangle encadrant, ...), ces données sont exploitées dans le module 180 par un logiciel de classement qualitatif ou de débit optimisé. On obtient ainsi en sortie du module 180, à l'étape 181, des ordres de classement des objets par qualité ou des ordres de découpe, le processus de fabrication pouvant être piloté en fonction des caractéristiques du produit analysé prédédemment.

Les circuits de traitement et d'interface mis en oeuvre selon la présente invention peuvent être commandés et gérés à partir d'un microordinateur de type PC.

On a représenté, sur la figure 12, un bus 272 de microordinateur de type PC, des circuits 271 d'interface avec ce bus 272 de type PC, des registres d'initialisation 261 reliés aux circuits d'interface 271. On a également représenté des circuits d'interface 251 pour l'accomplissement des fonctions réalisées au sein des modules 140,150, 160, 170.

Les circuits d'interface 251 permettent de réaliser l'interfaçage des données vers des cartes de processeur de traitement de signaux numériques. Les circuits d'interface 251 présentent troies voies de mémorisation "MEM" constituées par les trois voies couleurs R,V,B utilisées pour l'affichage écran, et quatre voies "TRAI" constituées par les quatre voies labellisées utilisées pour le traitement.

Le circuit 221 qui présente des liaisons avec les interfaces caméra 201 à 204, les circuits FIFO 211 à 214, le circuit 222 de correction d'éclairage, le circuit 231 de changement d'espace couleur et le circuit 241 de classifieur, comprend un ensemble de composants qui réalisent d'une part la gestion des signaux de contrôle de l'ensemble des circuits de la carte constituant le dispositif de traitement (génération des signaux d'horloge et des signaux de commande des autres blocs fonctionnels), d'autre part la gestion de la fonction "bitmap". Cette fonction "bitmap" permet, pour une ligne acquise d'un nombre N de pixels (par exemple N=2098 pour les cas de caméras couleur utilisables dans le cadre de la présente invention) de ne mémoriser et donc de ne traiter qu'un sous-ensemble de pixels déclarés utiles.

On peut remarquer que la mise en oeuvre de sources de lumière monochromatique 21 à 24 selon l'invention permet non seulement de visualiser et discriminer des particularités géométriques des pièces observées, mais également à titre complémentaire de régler chaque caméra 11 à 14 par rapport à une trace laser obtenue d'une source 21 à 24, ce qui permet d'améliorer l'alignement des caméras.

**Revendications**

1. Procédé de reconnaissance de particularités géométriques de pièces parallélépipédiques (5) de section polygonale comportant au moins une face secondaire (3) adjacente à une face principale (1), l'angle maximum défini entre la face principale considérée (1) et ladite face secondaire adjacente (3) étant compris entre 0° et 90°, caractérisé en ce qu'il comprend les étapes suivantes:

   a) disposer une source de lumière monochromatique (21) projetant un faisceau plan de rayonnement collimaté et monochromatique perpendiculairement à l'axe longitudinal de la pièce (5) de manière à éclairer de face ladite face secondaire (3) sans éclairer la surface plane de la face principale (1),

   b) disposer une caméra vidéo linéaire couleur (11) en regard de la face principale (1) considérée, de telle sorte que son axe soit perpendiculaire à ladite face principale (1) et qu'elle observe une ligne perpendiculaire à l'axe longitudinal de la pièce (5), dans le plan du faisceau de rayonnement collimaté et monochromatique, de manière à observer à la fois ladite face principale (1) et la projection de ladite face secondaire adjacente (3) sur la face principale (1),

   c) acquérir simultanément les trois signaux couleur R, V, B issus de la caméra (11), chaque signal représentant l'amplitude de l'une des trois composantes de base R, V, B en chaque point analysé suivant la ligne d'observation,

   d) traiter en temps réel les signaux R, V, B pour fournir une succession de pixels adjacents et associés selon l'appartenance de leurs composantes R, V, B à des domaines particuliers de l'espace R, V, B identifiés par des labels,

   e) produire un déplacement longitudinal relatif de la pièce à analyser par rapport à la caméra (11) et à la source de lumière monochromatique (21) pour observer l'ensemble de la face principale (1) et la projection de la face secondaire adjacente (3) sur ladite face principale (1), et

   f) reconnaître les particularités géométriques (8) de ladite face secondaire (3) à partir de l'identification des pixels correspondant à un domaine particulier de l'espace R, V, B identifié par un label correspondant à l'éclairage monochromatique de ladite source (21) de lumière monochromatique.

2. Procédé selon la revendication 1, caractérisé en ce que l'on dispose en outre une source (31) d'éclairage diffus à lumière polychromatique entre la caméra (11) et la face principale (1) de la pièce (5) pour éclairer de façon diffuse la ligne de la face principale (1) observée par la caméra (11), et en ce que l'on procède à une identification simultanée de défauts de structure ou de coloration de la face principale (1) et de particularités géométriques de la face secondaire adjacente (3), dans l'image captée par la caméra (11) et pré-traitée en temps réel à l'aide d'une opération de classification de pixels par couleur, chaque pixel de l'image captée étant associé à un sous-ensemble spécifique identifié par un label, ce qui permet de caractériser directement l'appartenance du pixel à une entité spécifique de l'image indépendamment de toute autre caractéristique de dimension ou de forme de la région d'image à laquelle appartient ledit pixel, et permet ainsi de détecter et discriminer d'une part lesdites particularités géométriques (8) de ladite face secondaire (3) et d'autre part des défauts (6, 7) de structure ou de coloration de la face principale (1) représentés par des pixels correspondant à des domaines particuliers de l'espace R, V, B identifiés par des labels ne correspondant pas audit éclairage monochromatique de la source (21) de lumière monochromatique.

3. Procédé selon la revendication 2, caractérisé en ce que lors de l'acquisition simultanée des trois signaux couleur R, V, B issus de la caméra linéaire (11), le traitement en temps réel des signaux R, V, B comprend un changement d'espace couleur réalisé électroniquement pour définir la couleur des points dans un nouvel espace tridimensionnel favorisant la discrimination des couleurs.

**4.** Procédé selon l'une quelconque des revendications 2 et 3, caractérisé en ce que lors de l'acquisition simultanée des trois signaux couleur R, V, B issus de la caméra linéaire (11), on corrige électroniquement en temps réel l'intensité des composantes R, V, B reçue par chaque pixel afin de compenser d'éventuelles hétérogénéités d'éclairage.

**5.** Procédé selon l'une quelconque des revendications 2 à 4, caractérisé en ce que l'opération de classification des pixels en sous-ensembles spécifiques munis d'un label selon la couleur s'effectue en une première phase préalable d'apprentissage des couleurs caractéristiques de chaque singularité à identifier dans l'image et à une deuxième phase de classification proprement dite en temps réel au cours de laquelle chaque pixel n'est plus caractérisé par un triplet de coordonnées dans un espace couleur, mais par le seul label auquel appartient la couleur correspondant à ce triplet de coordonnées.

**6.** Procédé selon l'une quelconque des revendications 2 à 5, caractérisé en ce que l'image couleur labellisée est soumise à une opération de filtrage, puis de segmentation de régions, afin de définir les attributs dimensionnels et géométriques de chaque singularité de structure ou de coloration et de chaque particularité géométrique présentes dans l'image captée par la caméra linéaire (14).

**7.** Procédé selon l'une quelconque des revendications 2 à 6, caractérisé en ce qu'il comprend une opération d'identification des défauts qui s'effectue en une première phase préalable d'apprentissage des défauts types et de détermination des paramètres les plus discriminants permettant d'identifier chaque type de défaut, et à une deuxième phase de reconnaissance des défauts en temps réel, au cours de laquelle il est déterminé un ensemble de paramètres décrivant chaque défaut extrait, et selon la valeur prise par chacun de ces paramètres, il est calculé la probabilité d'occurrence de chaque défaut préalablement appris et les types de défaut associés aux plus fortes probabilités d'occurrence sont alors considérés comme reconnus.

**8.** Procédé selon l'une quelconque des revendications 2 à 7, caractérisé en ce que l'on procède simultanément à la reconnaissance de particularités géométriques de plusieurs faces secondaires (3, 4) adjacentes à des faces principales (1, 2) et à une identification simultanée de défauts de structure ou de coloration desdites faces principales (1, 2) en disposant autant de sources de lumière monochromatique (21, 22) qu'il y a de faces secondaires (3, 4) et autant de caméras vidéo linéaires couleur (11, 12) qu'il y a de faces principales (1, 2), et en ce que lors de l'acquisition des signaux couleur R, V, B issus de chaque caméra linéaire (11, 12), on constitue des lignes d'image comprenant la juxtaposition de pixels issus des différentes caméras linéaires (11, 12).

**9.** Dispositif de reconnaissance de particularités géométriques de pièces parallélépipédiques (5) de section polygonale comportant au moins une face secondaire (3) adjacente à une face principale (1), l'angle maximum défini entre la face principale considérée (1) et ladite face secondaire (3) adjacente étant compris entre 0° et 90°, caractérisé en ce qu'il comprend au moins :

a) une source de lumière monochromatique (21) projetant un faisceau plan de rayonnement collimaté et monochromatique perpendiculairement à l'axe longitudinal de la pièce (5) de manière à éclairer de face ladite face secondaire (3) sans éclairer la surface plane de la face principale (1),

b) une caméra vidéo linéaire couleur (11) située en regard de la face principale (1) considérée, de telle sorte que son axe soit perpendiculaire à ladite face principale (1) et qu'elle observe une ligne perpendiculaire à l'axe longitudinal de la pièce (5), dans le plan du faisceau de rayonnement collimaté et monochromatique,

c) des moyens de support et d'entraînement pour provoquer un mouvement relatif entre d'une part la caméra (11) et la source de lumière monochromatique (21) et d'autre part la pièce (5) dans le sens de l'axe longitudinal de celle-ci de telle manière que des portions successives de la face principale (1) et de la face secondaire (3) de la pièce (5) sont respectivement observées et éclairées par la caméra (11) et la source (21) de lumière monochromatique,

d) des moyens électroniques d'acquisition et de traitement en temps réel des signaux couleur R, V, B délivrés par la caméra linéaire (11), pour fournir une succession de pixels adjacents et associés selon l'appartenance de leurs composantes R, V, B à des domaines particuliers de l'espace R, V, B identifiés par des labels, et permettre la reconnaissance des particularités géométriques (8) de ladite face secondaire (3) à partir de l'identification des pixels correspondant à un domaine particulier de l'espace R, V, B identifié par un label correspondant à l'éclairage monochromatique de ladite source (21) de lumière monochromatique.

**10.** Dispositif selon la revendication 9, caractérisé en ce qu'il comprend en outre une source (31) d'éclairage diffus à lumière polychromatique interposée entre la caméra (11) et la face principale (1) de la pièce (5) pour éclairer de façon diffuse la ligne de la face principale (1) observée par la caméra (11) et en ce que les moyens électroniques d'acquisition et de traitement en temps réel des signaux couleur R, V, B comprennent des moyens de discrimination pour détecter et discriminer d'une part lesdites particularités géométriques (8) de ladite face secondaire (3) et d'autre part des défauts (6, 7) de structure ou de coloration de la face principale (1).

**11.** Dispositif selon la revendication 10, caractérisé en ce que la source (31) d'éclairage diffus à lumière polychromatique comprend des première et deuxième sources lumineuses (308, 309) disposées transversalement par rapport à la pièce (5) à observer, de part et d'autre du plan contenant les axes de la caméra (11) et de la source de lumière monochromatique (21), des réflecteurs (302, 303) destinés à orienter la lumière polychromatique émise par lesdites première et deuxième sources lumineuses (308, 309) vers la ligne transversale observée par la caméra (11) et un carter (300) de support des sources lumineuses (308, 309) et des réflecteurs (302, 303), lequel carter comprend une ouverture transversale en forme de fente (301) permettant à la caméra (11) d'observer la ligne constituée par l'intersection entre les faisceaux de lumière polychromatique et la face principale (1) de la pièce (5).

**12.** Dispositif selon l'une quelconque des revendications 9 à 11, caractérisé en ce que la source de lumière monochromatique (21) comprend un générateur laser.

**13.** Dispositif selon l'une quelconque des revendications 9 à 11, caractérisé en ce que la source de lumière monochromatique (21) comprend une source de lumière polychromatique à laquelle est adjoint un filtre coloré.

**14.** Dispositif selon l'une quelconque des revendications 9 à 13, caractérisé en ce que la source de lumière monochromatique (21) comprend en outre un dispositif optique ou mécanique pour engendrer un faisceau lumineux plan perpendiculaire à l'axe de la pièce (5) et se projetant dans le plan contenant le centre optique de la caméra (11) et la ligne observée sur la surface analysée.

**15.** Dispositif selon l'une quelconque des revendications 9 à 14, appliqué à des pièces parallélépipédiques de section rectangulaire présentant deux faces principales opposées (1, 2) et deux faces secondaires ou rives (3, 4), caractérisé en ce qu'il comprend selon un premier plan perpendiculaire à l'axe de la pièce (5) des première et deuxième caméras (11, 12) vidéo linéaires couleur disposées en regard respectivement des première et deuxième faces principales opposées (1, 2) de la pièce (5), des première et deuxième sources (21, 22) de lumière monochromatique situées en regard respectivement des première et deuxième faces secondaires opposées (3, 4), des première et deuxième sources de lumière polychromatique (31, 32) interposées entre les première et deuxième caméras (11, 12) et les première et deuxième faces principales opposées (1, 2) de la pièce (5) et des moyens électroniques d'acquisition et de traitement en temps réel des signaux couleur R, V, B délivrés par les deux caméras linéaires (11, 12).

**16.** Dispositif selon la revendication 15, caractérisé en ce qu'il comprend en outre selon un deuxième plan perpendiculaire à l'axe de la pièce (5) et décalé longitudinalement par rapport audit premier plan perpendiculaire à l'axe de la pièce (5), des troisième et quatrième caméras vidéo linéaire couleur (13, 14) disposées en regard respectivement des première et deuxième faces secondaires opposées (3, 4) de la pièce (5), des troisième et quatrième sources (23, 24) de lumière monochromatique situées en regard respectivement des première et deuxième faces principales opposées (1, 2), des troisième et quatrième sources de lumière polychromatique (33, 34) interposées entre les troisième et quatrième caméras (13, 14) et les première et deuxième faces secondaires opposées (3, 4) et des moyens électroniques d'acquisition et de traitement en temps réel des signaux couleur R, V, B délivrés par l'ensemble des quatre caméras linéaires (11, 12, 13, 14).

**17.** Application du procédé selon l'une quelconque des revendications 2 à 8 et du dispositif selon l'une quelconque des revendications 10 à 16 à la détection de défauts de structure et de défauts de coloration présents sur une face plane d'une pièce de bois ainsi que simultanément à la détection de défauts ou de particularités géométriques affectant les deux rives adjacentes à la face plane observée.

FIG.1

FIG.5

FIG.3

FIG.2

FIG.4

FIG.6

FIG.7A   FIG.7B   FIG.7C   FIG.7D

# FIG.8

# FIG.9

# FIG.10

# FIG.10A

# FIG.11

| | |
|---|---|
| DÉFINITION DU MATÉRIAU : BOIS D'ŒUVRE | 101 |
| MODULE D'ACQUISITION COULEUR | 110 |
| PRODUCTION D'UNE IMAGE RVB | 111 |
| MODULE DE CHANGEMENT D'ESPACE COULEUR | 120 |
| NOUVELLES COORDONNÉES D'IMAGE | 121 |
| MODULE DE CLASSIFICATION DES PIXELS PAR COULEUR | 130 |
| IMAGE COULEUR LABELLISÉE | 131 |
| MODULE DE FILTRAGE | 140 |
| IMAGE LABELLISÉE FILTRÉE | 141 |
| MODULE DE SEGMENTATION | 150 |
| DÉFINITION DES RÉGIONS ET DE LEURS ATTRIBUTS | 151 |
| MODULE DE CLASSIFICATION PROBABILISTIQUE | 160 |
| RELEVÉ DES DÉFAUTS DÉTECTÉS | 161 |
| MODULE D'INTERPRÉTATION DES DÉFAUTS | 170 |
| LISTE DES DÉFAUTS FILTRÉS | 171 |
| MODULE D'OPTIMISATION OU DE GRADATION | 180 |
| ORDRE DE TRONÇONNAGE OU PLANCHES CLASSÉES PAR QUALITÉ | 181 |

132 APPRENTISSAGE →

142 APPRENTISSAGE →

162 APPRENTISSAGE →

172 RÈGLES DE DÉCISION →

182 RÈGLES DE DÉCOUPE OU RÈGLES DE GRADATION →

# FIG.12

**201** INTERFACE CAMÉRA  **202** INTERFACE CAMÉRA  **203** INTERFACE CAMÉRA  **204** INTERFACE CAMÉRA

FIFO W R  **211**
FIFO W R  **212**
FIFO W R  **213**
FIFO W R  **214**

**221** CONTRÔLE BITMAP

CORRECTION D'ÉCLAIRAGE  **222**

CHANGEMENT ESPACE COULEUR  **231**

CELLULE CODEUR

CLASSIFIEUR  **241**

**261** REGISTRES INITIALISATION

**251** INTERFACE PROCESSEUR  MEM  TRAI

INTERFACE BUS PC  **271**

BUS PC  **272**

**Office européen des brevets**

# RAPPORT DE RECHERCHE EUROPEENNE

Numero de la demande

EP 95 40 1649

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | US-A-4 831 545 (FLOYD STANLEY L ET AL) 16 Mai 1989<br>* colonne 7, ligne 41 - colonne 8, ligne 6; figure 12 *<br>--- | 1 | G01N33/46<br>G01N21/89 |
| A | WO-A-93 25894 (GRECON GRETEN GMBH & CO KG ;GRETEN ERNST (DE); MASSEN ROBERT (DE);) 23 Décembre 1993<br>* page 6, ligne 14 - page 7, ligne 15; figures *<br>--- | 1 | |
| A | DE-A-40 33 485 (AUTOSORT AB) 2 Mai 1991<br>* colonne 10, ligne 63 - colonne 12, ligne 14; figures *<br>--- | 1 | |
| A | WO-A-90 11488 (VISIONSMART INC) 4 Octobre 1990<br>* revendications 1-10 *<br>--- | 1 | |
| A | EP-A-0 320 391 (CENTRE TECHN BOIS AMEUBLEMENT) 14 Juin 1989<br>* le document en entier *<br>----- | 1 | DOMAINES TECHNIQUES RECHERCHES (Int.Cl.6)<br><br>G01N |

Le présent rapport a été établi pour toutes les revendications

| Lieu de la recherche | Date d'achèvement de la recherche | Examinateur |
|---|---|---|
| LA HAYE | 2 Octobre 1995 | Hoekstra, F |